# EUROPÄISCHE PATENTANMELDUNG

(11) **EP 4 606 397 A1**
(43) Veröffentlichungstag der Anmeldung: **27.08.2025**
(21) Anmeldenummer: 24159180.9
(22) Anmeldetag: 22.02.2024
(51) Int. Cl.: A61L 31/02, A61L 31/14, A61L 31/16, A61F 2/24

(54) **IMPLANTAT ZUR MEDIZINISCHEN VERSORGUNG VON MENSCHEN UND/ODER TIEREN SOWIE BIORESORBIERBARES VERBUNDMATERIAL HIERFÜR**

(71) Anmelder: Medizinische Universität Wien, 1090 Wien (AT); RHP-Technology GmbH, 2444 Seibersdorf an der Leitha (AT)
(72) Erfinder: Podesser, Bruno, 1220 Wien (AT); Bergmeister, Helga, 1030 Wien (AT); Enayati, Marjan, 3420 Klosterneuburg (AT); Bammer, Manfred, 1220 Wien (AT); Weber, Lukas, 1110 Wien (AT); Sajti, Laszlo, 2700 Wiener Neustadt (AT); Horky, Jelena, 1070 Wien (AT); Fedel, Mariangela, 38042 Baselga di Pinè (IT); Grasl, Christian, 1210 Wien (AT)
(74) Vertreter: Wirnsberger & Lerchbaum Patentanwälte OG

(57) **Zusammenfassung**

Die Erfindung betrifft ein Implantat zur medizinischen Versorgung von Menschen und/oder Tieren. Um ein Implantat bereitzustellen, das sich im Körper während und/der nach dem Heilungsprozess auflöst und gleichzeitig möglichen Entzündungen nach einer Operation entgegenwirkt, umfasst oder besteht das Implantat aus:
a) einem bioresorbierbaren Implantatgrundkörper;
b) einem bioresorbierbaren Befestigungskörper, der mit dem Implantatgrundkörper in Verbindung steht und über welchen der Implantatgrundkörper in einem menschlichen oder tierischen Körper befestigbar ist;
c) eine antibakteriell wirkende Substanz.

Des Weiteren betrifft die Erfindung ein bioresobierbares Verbundmaterial für ein derartiges Implantat.

## Beschreibung

Die Erfindung betrifft ein Implantat zur medizinischen Versorgung von Menschen und/oder Tieren.

Des Weiteren betrifft die Erfindung ein bioresorbierbares Verbundmaterial für ein derartiges Implantat.

Die moderne Operationstechnik kann zur medizinischen Versorgung von Menschen und/oder Tieren für eine Anwendung in Bereichen wie kardiovaskuläre Krankheiten bis hin zu Knochenbrüchen auf eine Vielzahl diverser Implantate zurückgreifen. Das Spektrum möglicher Implantate ist sehr breit und kann beispielsweise körperähnliche Implantate in Form von Allografts für Rupturen oder insbesondere Rerupturen von Kreuzbändern umfassen. Häufig bestehen Implantate jedoch aus körperfremden Materialien wie Keramiken, beispielsweise im Bereich der Zahnchirurgie, oder insbesondere Metalllegierungen, beispielsweise Stents zur Versorgung von Herzkrankheiten. Für Knochenschrauben, Knochennägel und Knochenplatten kommen häufig Titanlegierungen zum Einsatz, beispielsweise zur Heilung eines Oberschenkelhalsbruches. Nach einem Heilungsprozess werden die entsprechenden Implantate, sofern notwendig und aus medizinischer Sicht sinnvoll und vom Risiko-Nutzen-Verhältnis machbar, grundsätzlich wieder aus dem Körper entfernt. Für die Zeit des Heilungsprozesses sollen die Materialien für die Implantate so ausgelegt sein, dass diese neben einer Biokompatibilität, zum Beispiel bei Kontakt mit Blut, vor allem ein vorgegebenes mechanisches Beanspruchungsprofil erfüllen müssen, damit es während des Heilungsprozesses nicht zu weiteren Komplikationen aufgrund von Materialversagen kommt.

Neben den insbesondere für die Versorgung von Frakturen bekannt gewordenen Implantaten aus korrosionsbeständigen Materialien wie Titanlegierungen sind auch Implantate bekannt geworden, welche sich im Laufe der Zeit im menschlichen Körper auflösen. Ein gesonderter operativer Schritt für eine Implantatentfernung kann dann, sofern dieser überhaupt möglich wäre, entfallen. Hierfür eignen sich insbesondere Magnesiumlegierungen, wobei viele Jahre Magnesiumlegierungen mit merklichen Anteilen an Metallen der Seltenen Erden im Vordergrund standen. Derartige Magnesiumlegierungen lösen sich im Laufe der Zeit im menschlichen Körper auf; allerdings sind solche Legierungen so korrosionsbeständig, dass die Implantate nicht bereits während eines Heilungsprozesses derart weitgehend von der Körperflüssigkeit zersetzt werden, dass die Implantate während des Heilungsprozesses an der erforderlichen Funktionalität verlieren. Derartige Implantate können beispielsweise auch zur Versorgung von Frakturen eingesetzt werden, weil der Kontakt mit Körperflüssigkeit nicht so intensiv ist, dass eine sehr schnelle Korrosion erfolgen kann.

Bei den sich im Körper im Laufe der Zeit auflösenden Magnesiumlegierungen mit Anteilen an zulegierten Metallen der Seltenen Erden bestehen medizinische Bedenken hinsichtlich der zulegierten Metalle der Seltenen Erden, da diese nach der Auflösung eines Implantates im Körper verbleiben. Langzeitstudien hierzu scheint es noch nicht zu geben, die Bedenken sind jedoch erheblich, weil die Konzentrationen der Metalle der Seltenen Erden in den verwendeten Magnesiumlegierungen erheblich sein können. Die Legierung WE43, eine Standardlegierung für Implantate, weist beispielsweise etwa 4 Gew.-% Yttrium und 3 Gew.-% weitere Metalle der Seltenen Erden (zum Beispiel Neodym und/oder Gadolinium) auf. Insbesondere wenn derartige Implantate in Bereichen eingesetzt werden, in welchen die Implantate sehr stark mit Körperflüssigkeit in Kontakt kommen, beispielsweise als kardiovaskuläre Stents, gelangen die Metalle der Seltenen Erden aus derartigen Implantaten unkontrolliert in den Blutkreislauf und es bestehen Bedenken, dass dies zu Spätfolgen führen könnte.

Man ist daher dazu übergegangen, bei im Körper abbaubaren Implantaten den Anteil an Metallen der Seltenen Erden zu reduzieren. Mittlerweile wurden für die Behandlung von Frakturen auch Legierungen entwickelt, welche im Wesentlichen frei von Metallen der Seltenen Erden ausgebildet sind (P. Holweg et al., A lean bioabsorbable magnesium-zinc-calcium alloy ZX00 used for operative treatment of medial malleolus fractures, Bone Joint Res 2020, 9(8), 477; P. Holweg et al., A lean magnesium-zinc-calcium alloy ZX00 used for bone fracture stabilization in a large growing-animal model, Acta Biomaterialia 113 (2020) 646).

Bei der Entwicklung bioresorbierbarer Implantate ergibt sich ein sehr spezifisches Anforderungsprofil an das mittels Operation einzusetzende Implantat: Zunächst soll das Implantat erwarteten mechanischen Belastungen über die Dauer des erwarteten Heilungsprozesses Stand halten können. Verliert das Implantat beispielsweise während des Heilungsprozesses zu stark an Festigkeit, kann dies für den Heilungsprozess problematisch sein. Darüber hinaus sollte sich das Implantat im Idealfall zumindest merklich erst nach dem Heilungsprozess auflösen. Ein vorzeitiges Auflösen kann den Heilungsprozess beeinträchtigen. Es hängt letztlich auch damit zusammen, dass mit der Auflösung des Implantats auch eine Abnahme der mechanischen Kennwerte desselben einhergeht. Insbesondere bei Implantaten, die sehr stark in Kontakt mit Körperflüssigkeiten stehen, insbesondere Blut, erweist es sich als äußerst schwierig, hierfür ein geeignetes Material anzugeben. Dies betrifft beispielsweise am oder im Herz eines Menschen eingesetzte Implantate, beispielsweise Stents. Insbesondere bei einem engen Kontakt des Implantats mit Körperflüssigkeit wie Blut kommt noch die erhöhte Gefahr einer bakteriellen Infektion infolge der Operation hinzu. Dies gilt umso mehr, wenn neben dem eigentlichen Implantatgrundkörper zusätzliche Komponenten zur Positionierung des Implantatgrundkörpers vorgesehen sind, wie dies beispielsweise bei Annuloplastieringen zur Versorgung von Herzklappenerkrankungen, erforderlich ist.

Ausgehend vom Stand der Technik stellt sich die Erfindung die Aufgabe, ein Implantat der eingangs genannten Art derart weiterzubilden, dass dieses auch bei intensivem Kontakt mit Körperflüssigkeit, insbesondere Blut, während eines Heilungsprozesses ausreichende Stabilität aufweist, sich aber spätestens nach einem Heilungsprozess im Wesentlichen vollständig im Körper auflöst und gleichzeitig insbesondere im Anschluss an eine Operation einer bakteriellen Infektion entgegenwirkt.

Ein weiteres Ziel der Erfindung ist es, in diesem Zusammenhang ein hierfür geeignetes Verbundmaterial anzugeben.

Die Aufgabe der Erfindung wird gelöst, wenn ein Implantat der eingangs genannten Art folgende Komponenten umfasst oder aus diesen besteht:
a) einen bioresorbierbaren Implantatgrundkörper;
b) einen bioresorbierbaren Befestigungskörper, der mit dem Implantatgrundkörper in Verbindung steht und über welchen der Implantatgrundkörper in einem menschlichen oder tierischen Körper befestigbar ist;
c) eine antibakteriell wirkende Substanz.

Mit einem erfindungsgemäßen Implantat werden mehrere Vorteile erreicht: Zum einen ist der Implantatgrundkörper ebenso wie der Befestigungskörper, welcher mit dem Implantatgrundkörper in Verbindung steht und über welchen der Implantatgrundkörper im menschlichen oder tierischen Körper befestigbar ist, bioresorbierbar. Bioresorbierbar bedeutet hierbei, dass sich der Implantatgrundkörper ebenso wie der Befestigungskörper hierfür zu mindestens im Wesentlichen vollständig im Laufe der Zeit im menschlichen Körper auflöst. Die Auflösung im menschlichen Körper ist dabei sowohl für den Implantatgrundkörper als auch für den Befestigungskörper so abgestimmt, dass ein Heilungsprozess nicht gefährdet ist. Die zusätzlich vorgesehene antibakteriell wirkende Substanz verhindert oder verringert zumindest das Risiko einer bakteriellen Infektion im Bereich des Implantates vor allem unmittelbar nach einer Operation und dem Einsetzen des Implantats. Die antibakteriell wirkende Substanz ist Bestandteil des Implantats und wird daher aufgrund der Auflösung desselben entsprechend freigesetzt. Da die antibakteriell wirkende Substanz in der Regel nicht nur innerhalb einer der Komponenten (Implantatgrundkörper und/oder Befestigungskörper) vorliegen kann, sondern auch an der jeweiligen Oberfläche der Komponente präsent ist, tritt die antibakterielle Wirkung unmittelbar nach Einsetzen des Implantats ein. Mit dem Abbau des Implantats erfolgt dann die kontinuierliche Freisetzung der antibakteriell wirkenden Substanz, soweit diese im Inneren einer der Komponenten vorliegt. Damit kann über die gesamte Zeit der Präsenz eines Implantats im Körper die antibakterielle Wirkung aufrechterhalten werden.

Die Bioresorbierbarkeit des Implantatkörpers und des Befestigungskörpers sind aufeinander je nach Einsatzzweck abgestimmt. Wenn der Implantatgrundkörper nach der Befestigung des Implantats im menschlichen Körper im Grundsatz auch selbstständig in einer fixierten Position verbleibt, kann der Befestigungskörper aus einem Material gefertigt sein, welches sich rascher als der Implantatgrundkörper im Körper auflöst. Ist hingegen die Befestigung während des gesamten Heilungsprozesses erforderlich und dient der Befestigungskörper auch dazu, den Implantatgrundkörper in einer fixierten Position zu halten, sollte sich der Befestigungskörper langsamer als der Implantatgrundkörper auflösen. Idealerweise löst sich dann der Befestigungskörper erst dann merklich auf, wenn der Heilungsprozess abgeschlossen ist, also wenn der Implantatgrundkörper nicht mehr erforderlich ist, und der Befestigungskörper bis dahin seine Funktion als Fixiermittel für den Implantatgrundkörper erfüllen kann. Die Auflösung des Befestigungskörpers kann in diesem Fall noch vor Abschluss des Heilungsprozesses beginnen, darf aber nicht soweit fortschreiten, dass der Implantatgrundkörper bereits vor Beendigung des Heilungsprozesses nicht mehr selbstständig positionsstabil gehalten werden kann oder gehalten wird.

Der Implantatgrundkörper ist mit Vorteil aus einem Metall, einem Verbundwerkstoff mit überwiegend metallischen Anteilen oder insbesondere einer Metalllegierung gebildet. Es kann insbesondere auch vorgesehen sein, dass der Implantatgrundkörper aus einer Magnesiumlegierung gebildet ist. Dabei kann die Magnesiumlegierung im Masseprozent folgende Elemente aufweisen oder aus diesen bestehen:
0,2 % bis 1,0 %, bevorzugt 0,3 % bis 0,8 %, insbesondere 0,5 % bis 0,7 %, Zink;
0,2 % bis 1,0 %, bevorzugt 0,3 % bis 0,7 %, insbesondere 0,4 % bis 0,6 %, Calcium; optional weitere Legierungselemente wie Titan, Bor oder Silicium im Ausmaß von insgesamt weniger als 0,1 %, bevorzugt weniger als 0,05 %;
Rest Magnesium und herstellungsbedingte Verunreinigungen.

Magnesiumlegierungen sind besonders geeignet, um bioresorbierbar ausgebildet zu sein und damit vom Körper abgebaut werden zu können. Gleichzeitig bringen Magnesiumlegierungen für viele Implantatanwendungen ausreichende mechanische Kennwerte mit, insbesondere zur Versorgung von Herzkrankheiten. In diesem Zusammenhang erweist es sich besonders günstig, wenn die Magnesiumlegierung im Massenprozent 0,2 % bis 1,0 %, bevorzugt 0,3 % bis 0,8 %, insbesondere 0,5 % bis 0,7 %, Zink und gleichzeitig 0,2 % bis 1,0 %, bevorzugt 0,3 % bis 0,7 %, insbesondere 0,4 % bis 0,6 %, Calcium aufweist. Dabei ist mit Vorteil vorgesehen, dass ein Zinkanteil höher als ein Calciumanteil in der Legierung ist. Zink trägt insbesondere zur Festigkeit bei, wohingegen Calcium insbesondere auch zur Korrosionsbeständigkeit beiträgt, sodass eine gewisse Haltbarkeit auch bei Kontakt mit korrosiven Medien wie Körperflüssigkeit, insbesondere Blut, gegeben ist. Im Vergleich mit Legierungen aus dem Stand der Technik, welche niedrigere Gehalte an Zink und Calcium und ein Verhältnis von Zink zu Calcium von 1:1 aufweisen, kann durch ein abgestimmtes Legierungskonzept insbesondere für Herzimplantate ein ausgewogenes Eigenschaftsprofil aus ausreichender Haltbarkeit unter Erhalt vorgegebener Festigkeiten einerseits und einer anschließenden Degradierung andererseits gefunden werden. Besonders bevorzugte Gehaltsbereiche liegen dabei in Massenprozent für Zink im Bereich von 0,55 % bis 0,70 % und für Calcium im Bereich von 0,50 % bis 0,68 %. Daneben können optional weitere Legierungselemente wie Titan, Bor oder Silicium im Ausmaß von insgesamt weniger als 0,1 %, bevorzugt weniger als 0,05 % vorliegen. Der Rest wird aus Magnesium und herstellungsbedingten Verunreinigungen gebildet. Ein Gehalt an herstellungsbedingten Verunreinigungen liegt üblicherweise unter 0,01 %, bevorzugt unter 0,005 %.

Es ist auch von Vorteil, wenn die Magnesiumlegierung mit Ausnahme von herstellungsbedingten Verunreinigungen frei von Metallen der Seltenen Erden ist. Wie erwähnt betragen die herstellungsbedingten Verunreinigungen bevorzugt weniger als 0,005 %. Bezogen auf die Metalle der Seltenen Erden betragen die entsprechenden herstellungsbedingten Verunreinigungen besonders bevorzugt in Massenprozent weniger als 0,001 %, besonders bevorzugt weniger als 0,0005 %.

Ein Gehalt an Eisen sollte weniger als 100 ppm, vorzugsweise weniger als 50 ppm betragen, damit eine Korrosion der Magnesiumlegierung nicht beschleunigt wird.

Eine Form des Implantatgrundkörpers richtet sich nach dem jeweiligen Anwendungszweck. Der Implantatgrundkörper kann beispielsweise als Rohr ausgebildet sein. Möglich ist es auch, insbesondere für Ringe, Nägel, Drähte oder dergleichen, wenn der Implantatgrundkörper aus einem Vollmaterial gefertigt ist, insbesondere einer Stange, vorzugsweise einer extrudierten Stange, die gegebenenfalls durch Equal Channel Angular Pressing (ECAP) ein- oder mehrfach umgeformt ist. Für diverse Durchmesser ergibt sich bei der Fertigung aus einer Stange, insbesondere einer extrudierten Stange, ein homogenes Gefüge. Ein derartiges Gefüge kann durch ECAP und auch in Bezug auf Festigkeiten noch weiter verbessert werden, sofern dies gewünscht ist. Letztlich hängt dies von den gewünschten mechanischen Eigenschaften und Korrosionseigenschaften ab, da keine Schritte vorgenommen werden müssen, welche das Material noch besser gestalten als unbedingt üblich. Bei kleinen Durchmessern kann auch ein Ziehprozess angewendet werden, wobei ein zuvor extrudiertes Stangenmaterial beispielsweise als Ausgangsmaterial dienen kann. Mit Drahtziehen können Implantate mit einem Durchmesser von weniger als 5 mm, insbesondere weniger als 3 mm, erstellt werden. Auch andere Prozesse wie ein Hämmern können angewendet werden, wenn kleine Durchmesser eines Implantates erreicht werden sollen.

Für viele Anwendungen ist es ausreichend, wenn der Implantatgrundkörper im Wesentlichen mit einem konstanten Querschnitt ausgebildet ist. Dabei kann der Grundkörper, wenn dieser aus einem Grundmaterial wie einer Stange erstellt ist, auch Umformprozessen unterzogen worden sein, beispielweise einem Biegen. Möglich ist es auch, dass der Implantatgrundkörper aus einem Halbmaterial durch spannabhebende Bearbeitung, beispielsweise Fräsen oder Drehen herausgearbeitet ist.

Die Vorzüge der Erfindung kommen besonders deutlich zum Tragen, wenn der Implantatgrundkörper ein Annuloplastiering ist, beispielsweise ein Mitralring, insbesondere ein offener Mitralring, oder ein Tricuspidalring. Ein Mitralring kann zum Beispiel mit dem Befestigungskörper im Herz eines Menschen oder gegebenenfalls Tieres eingesetzt werden. Dabei wird das Implantat über den vorgesehenen Befestigungskörper entsprechend befestigt. Der Mitralring selbst ist etwa ringförmig geformt und kann dabei offen ausgebildet sein, also als nicht geschlossener Ring. Dies erlaubt es insbesondere, den Mitralring auf besonders einfache Weise aus einer abgelängten Stange zu formen, wobei das abgelängte Stangenteil nach der Ablängung gebogen wird, um den Mitralring zu bilden. Anschließend wird der Mitralring mit dem Befestigungskörper kombiniert, wobei das Implantat zusätzlich die antibakteriell wirksame Substanz umfasst und in dieser Konstellation operativ eingesetzt werden kann.

Zur Optimierung der Eigenschaften kann der Implantatgrundkörper wärmebehandelt sein. Eine Wärmebehandlung kann beispielsweise von Vorteil sein, wenn der Implantatgrundkörper, insbesondere ein Annuloplastiering wie ein Mitralring oder ein Tricuspidalring, aufgrund des Herstellungsprozesses hohe innere Spannungen aufweist. Beispielsweise kann der Implantatgrundkörper aus einer Magnesiumlegierung gebildet sein, die wärmebehandelt ist, wobei vorzugsweise eine Härte von zumindest 55 HV2, vorzugsweise zumindest 60 HV2, und/oder eine Zugfestigkeit zumindest 200 MPa beträgt. Höhere Werte HV2 von zumindest 65 HV2 und/oder Zugfestigkeiten von mehr als 230 MPa oder mehr als 250 MPa sind ebenso möglich.

Der Begriff des Befestigungskörpers ist im Rahmen der gegenständlichen Offenbarung breit zu verstehen. Der Befestigungskörper kann beispielsweise einen Faden darstellen, mit dem der Implantatgrundkörper beispielsweise an einem umliegenden Gewebe, beispielsweise im Herzen, oder einem Knochenteil fixiert wird. Der Befestigungskörper kann aber auch beispielsweise als flexibles oder starres Netz ausgebildet sein, welches ganz oder teilweise mit dem Implantatgrundkörper in Verbindung steht. Bevorzugt ist der Befestigungskörper biegsam ausgebildet. Insbesondere weist der Befestigungskörper eine größere Biegsamkeit als der Implantatgrundkörper auf.

Mit Vorteil nimmt der Befestigungskörper den Implantatgrundkörper vollständig oder zumindest überwiegend auf, insbesondere wenn es sich um einen Annuloplastiering, beispielsweise einen Mitralring handelt. Bei einem Mitralring wird der Befestigungskörper über den Implantatgrundkörper geschoben, sodass der Mitralring über den Befestigungskörper an der entsprechenden Position im Herz befestigt werden kann. Um ein entsprechendes Aufnehmen des Implantatgrundkörpers zu ermöglichen kann der Befestigungskörper allgemein rohrförmig ausgebildet sein.

In Bezug auf die geforderte Bioresorbierbarkeit und Biegsamkeit ist es günstig, wenn der Befestigungskörper aus einem Polymer gebildet ist. Das Polymer kann so gestaltet sein, dass sich dieses abgestimmt auf die Auflösung des Implantatgrundkörpers ebenso im Körper auflöst. Dabei kann der Befestigungskörper aus einem Polymer gebildet sein, das durch Elektrospinnen abgeschieden ist. Durch Elektrospinnen bilden sich Mikro- und/oder Nanofasern, also Fasern mit einer bestimmten Länge und/oder einem Durchmesser im Bereich von Mikrometern bis hin zu wenigen Nanometern. Es hat sich gezeigt, dass ein entsprechend abgeschiedener Befestigungskörper insbesondere in Bezug auf eine Aufnahme der antibakteriell wirkenden Substanz und deren spätere Freisetzung im Körper von Vorteil sein kann.

Von besonderem Vorteil ist es, wenn der Befestigungskörper aus oder mit einem Polycaprolacton oder einem Derivat davon gebildet ist. Polycaprolacton weist einen relativ niedrigen Schmelzpunkt von 60 °C bei einem mittleren Molekulargewicht Mn von 80 kDa auf. Dies erlaubt es, Polycaprolacton auf verschiedene Arten unter Formgebung zu verarbeiten, beispielsweise durch Gießen, Tropfen oder das zuvor erwähnte Elektrospinnen.

Die antibakteriell wirkende Substanz umfasst mit Vorteil Nanopartikel oder besteht aus diesen. Nanopartikel sind dabei Partikel, die zumindest in einer Längendimension (x-, y- und/oder z-Richtung) einen durchschnittlichen Durchmesser von weniger als 100 nm aufweisen. Hierbei hat es sich insbesondere bewährt, wenn die antibakteriell wirkende Substanz Nanopartikel aus Silber enthält oder aus diesen besteht. Nanopartikel aus Silber können effektiv dafür sorgen, dass nach einer Operation bakterielle Infektionen im Bereich des Implantats verhindert oder zumindest die Wahrscheinlichkeit hierfür verringert wird. Dabei ist auch von Vorteil, dass die Nanopartikel bakterielle Membranen beschädigen und daher sowohl gegen grampositive als auch gramnegative Bakterien wirken.

Die im Rahmen der Erfindung eingesetzten Nanopartikel sind vorzugsweise durch Laserablation hergestellt. Des Weiteren können die Nanopartikel ultrarein vorliegen, also mit einer Reinheit von mehr als 99,999 %.

Die antibakteriell wirkende Substanz kann beispielsweise an der Oberfläche des Implantatgrundkörpers aufgebracht sein, beispielsweise durch direkte Beschichtung desselben. Bevorzugt ist es jedoch, wenn die antibakteriell wirkende Substanz in und/oder am Befestigungskörper verteilt ist, insbesondere in einem gesamten Volumen des Befestigungskörpers homogen verteilt ist. Dabei ist es besonders effektiv, wenn die antibakteriell wirkende Substanz gemeinsam mit einem Grundmaterial des Befestigungskörpers, beispielsweise ein Polymer wie Polycaprolacton, verarbeitet ist. Dadurch liegt die antibakteriell wirkende Substanz nicht nur homogen und agglomeratfrei im Befestigungskörper vor, sondern wird mit dessen Auflösung über lange Zeit in entsprechenden Dosen freigesetzt. Mit anderen Worten: Mit der Auflösung des bioresorbierbaren Grundkörpers erfolgt auch eine, insbesondere stetige, fortdauernde lokale Freisetzung der antibakteriell wirkenden Substanz, sodass nicht nur unmittelbar nach einer Operation, sondern auch im Verlauf eines Heilungsprozesses eine antibakterielle Wirkung gegeben ist.

Ist die antibakteriell wirkende Substanz in und/oder am Befestigungskörper verteilt vorgesehen, ist es von Vorteil, wenn diese in Massenprozent in einem Anteil von 0,10 % bis 6,0 %, bevorzugt 0,20 % bis 4,5 %, insbesondere 0,30 % bis 4,0 %, beispielsweise 0,50 % bis 1,5 %, bezogen auf die Gesamtmasse von antibakteriell wirkender Substanz und Befestigungskörper, vorliegt. Ein gewisser Mindestgehalt an antibakteriell wirkender Substanz ist erforderlich, um die gewünschte Wirkung zu erreichen. Allzu hohe Gehalte von antibakteriell wirkender Substanz können eher zu Komplikationen führen. Insofern sind mittlere Gehalte von beispielsweise (in Massenprozent) 0,20 % bis 1,0 % an antibakteriell wirkender Substanz besonders bevorzugt. Einerseits wird die gewünschte antibakterielle Wirkung erreicht, andererseits sind Komplikationen aus der Freisetzung der antibakteriellen Substanz, beispielsweise Nanopartikeln, minimiert.

Neben Nanopartikeln können alternativ oder ergänzend auch Antibiotika, insbesondere nicht oder schlecht wasserlösliche Antibiotika wie Rifampicin zum Einsatz kommen, da solche Antibiotika aufgrund der schlechten oder nicht gegebenen Wasserlöslichkeit erst im Laufe der Degradation des Befestigungskörpers freigesetzt werden.

Das weitere Ziel der Erfindung wird durch ein bioresorbierbares Verbundmaterial für ein Implantat, das im menschlichen oder tierischen Körper implantiert wird, bestehend aus oder umfassend eine bioresorbierbare Trägersubstanz und eine antibakteriell wirkende Substanz, wobei das Verbundmaterial elektrogesponnen ist und/oder als Beschichtung vorliegt, erreicht.

Es wurde erkannt, dass eine besonders effektive Freisetzung einer antibakteriell wirkenden Substanz in einem Verbundmaterial, welches im Zusammenhang mit Implantaten im menschlichen oder tierischen Körper verwendet wird, erreicht werden kann, wenn das Verbundmaterial elektrogesponnen ist und/oder als Beschichtung vorliegt. Durch Elektrospinnen lassen sich besonders feine Fäden im Nanometerbereich erstellen, zwischen welchen dann die antibakteriell wirkende Substanz vorliegt. In ähnlicher Weise können vorzugsweise besonders dünne Schichten von einigen µm an Herzklappen oder anderen insbesondere in einem Herz eingesetzten Implantaten zu einer raschen und während des Abbaus der bioresorbierbaren Trägersubstanz kontinuierlichen Freisetzung der antibakteriell wirkenden Substanz führen. Dabei kann die Beschichtung auch durch Elektrospinnen, 3-D-Druck oder Schäumen erzeugt sein. Insbesondere mittels Elektrospinnen lassen sich für das Verbundmaterial auch gute mechanische Eigenschaften hinsichtlich Festigkeit und Nähbarkeit verbunden mit einer Langzeitstabilität erreichen.

Bevorzugt ist es dabei, wenn die Trägersubstanz bei Temperaturen von weniger als 100 °C aufschmelzbar ist. Dadurch lässt sich auf relativ einfache Weise eine Verarbeitung des Verbundmaterials erreichen, zumal die Trägersubstanz in der Regel den überwiegenden Anteil darstellt. Dies trifft insbesondere zu, wenn die Trägersubstanz ein Polymer, insbesondere Polycaprolacton oder ein Derivat davon ist.

Die antibakteriell wirkende Substanz kann Nanopartikel umfassen oder aus diesen bestehen. Dabei kann die antibakteriell wirkende Substanz metallische Nanopartikel enthalten oder aus diesen ausschließlich bestehen. Die Nanopartikel können beispielsweise ausgewählt sein aus der Gruppe bestehend aus Silber, Kupfer, Zink und Magnesium. Die Nanopartikel werden bevorzugt durch Laserablation in einem geeigneten Lösungsmittel erzeugt, um eine hohe Reinheit der Nanopartikel zu erreichen. Mit Vorteil kommen Nanopartikel aus Silber zum Einsatz. Die Nanopartikel können auch direkt in einem gelösten Polymer oder einer Monomerlösung erzeugt werden.

Die antibakteriell wirkende Substanz kann in bioresorbierbarem Verbundmaterial in Massenprozent in einem Anteil von 0,10 % bis 6,0 %, bevorzugt 0,20 % bis 4,5 %, insbesondere 0,30 % bis 4,0 %, 0,50 % bis 1,5 %, bezogen auf die Gesamtmasse von antibakteriell wirkender Substanz und Trägersubstanz, vorliegen. Bevorzugte Anteile liegen im Bereich von etwa 0,15 % bis 1,75 %.

Weitere Merkmale, Vorteile und Wirkungen in Zusammenhang mit der gegenständlichen Offenbarung ergeben sich aus den nachfolgend dargestellten Ausführungsbeispielen. In den Zeichnungen, auf welche dabei Bezug genommen wird, zeigen:
Fig. 1 einen handelsüblichen Mitralring mit einer umgebenen Hülse;
Fig. 2 ein 3-D-Modell eines offenen Mitralringes;
Fig. 3 nicht wärmebehandelte Mitralringe aus verschiedenen Legierungen und in Abhängigkeit einer Zeitspanne in Kontakt mit simulierter Körperflüssigkeit;
Fig. 4 Härtewerte HV2 für zwei Legierungen in Abhängigkeit einer Wärmebehandlung;
Fig. 5 eine Abhängigkeit eines Durchmessers von wärmebehandelten und nicht wärmebehandelten Mitralringen in Abhängigkeit einer Dauer eines Kontaktes mit verdünnter Salzsäure;
Fig. 6 eine gleichbleibende Konzentration von Nanopartikeln aus Silber in einer Trägersubstanz vor und nach einer Verarbeitung der Trägersubstanz;
Fig. 7 eine elektrogesponnene Hülse auf einem Mitralring;
Fig. 8 und Fig. 9 rasterelektronenmikroskopische (REM-) Aufnahmen einer elektrogesponnenen Hülse;
Fig. 10 ein Diagramm zur Zugfestigkeit und Dehnung von Stangen aus einer Magnesiumlegierung des Typs ZX00 extrudiert auf verschiedene Durchmesser;
Fig. 11 ein Diagramm zu einer Dauerwechselbelastung einer Magnesiumlegierung des Typs ZX00 mit einer Spannungsamplitude über der Zykluszahl bis zum Bruch, der Testdauer und einer entsprechenden Implantationsdauer bei einer durchschnittlichen Herzschlagfrequenz;
Fig. 12 Ergebnisse von 3-Punkt-Biegeversuchen an nicht implantierten Pins aus Legierungen des Typs ZX00 und WE43;
Fig. 13 Ergebnisse von 3-Punkt-Biegeversuchen an Pins aus einer Legierung des Typs ZX00 vor (AR) und 22 Wochen nach Implantation;
Fig. 14 Ergebnisse von 3-Punkt-Biegeversuchen an Pins aus Legierungen des Typs ZX00 und WE43 vor (AR) und 22 Wochen nach einer Implantation;
Fig. 15 Vergleich von Kraft-Weg-Kurven aus 3-Punkt-Biegeversuchen von sterilisierten und nicht sterilisierten Pins des Typs ZX00;
Fig. 16 Ergebnisse von 3-Punkt-Biegeversuchen an Pins aus einer Legierung des Typs ZX00 nach verschiedenen Degradationszeiten in simulierter Körperflüssigkeit;
Fig. 17 in 3-Punkt-Biegeversuchen erreichte maximale Kraft für ZX00-Pins und WE43-Pins in Abhängigkeit einer Degradationszeit von bis zu 5 Wochen in simulierter Körperflüssigkeit;
Fig. 18 in 3-Punkt-Biegeversuchen erreichte Elastizitätsgrenze für ZX00-Pins und WE43-Pins in Abhängigkeit einer Degradationszeit von bis zu 5 Wochen in simulierter Körperflüssigkeit;
Fig. 19 Diagramm zur Lebensfähigkeit von Zellen bei unterschiedlichen Konzentrationen von Nanopartikeln aus Silber;
Fig. 20 Diagramm zum OD-Wert bei unterschiedlichen Konzentrationen von Nanopartikeln aus Silber;
Fig. 21 Röntgenbilder von je zwei Ratten mit je vier Pins aus Magnesiumlegierungen des Typs WE43 und ZX00 zu unterschiedlichen Zeitpunkten nach der Implantation;
Fig. 22 Fotos von explantierten WE43- und ZX00-Pins;
Fig 23 Lichtmikroskopische Aufnahmen von Pins aus Magnesiumlegierungen (WE43 und ZX00) und umliegendem Hautgewebe.

### 1. Herstellung von Mitralringen

Als Ausgangsmaterial für bioresorbierbare Mitralringe wurden die Legierungen Mg-0,6Zn-0,5Ca (im Folgenden bezeichnet als: ZX00) definiert und als extrudiertes Stangenmaterial mit 6 und 12 mm Durchmesser eingesetzt. Als Vergleichsmaterial wurde die kommerziell verfügbare Magnesiumbasislegierung WE43 verwendet.

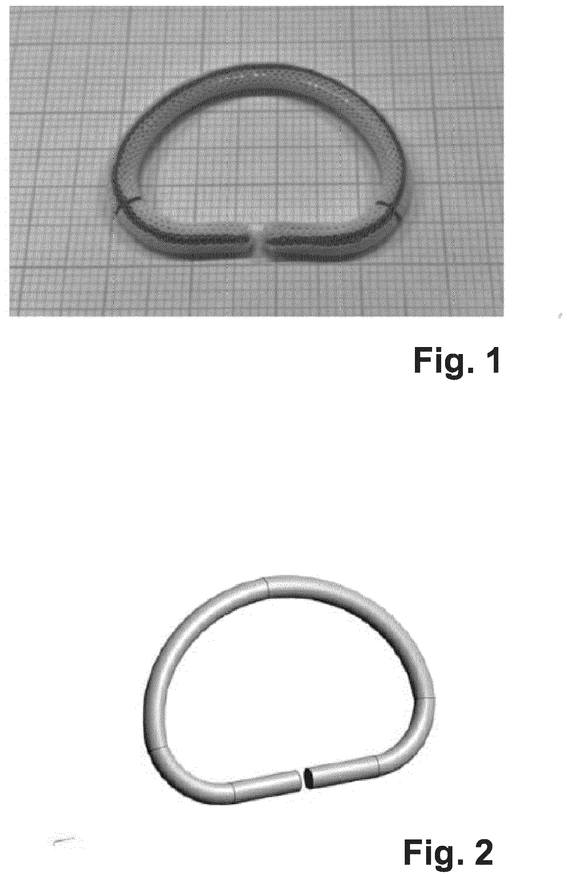

Bei den Designüberlegungen für die Mitralringe wurden geschlossene und offene Ringe mit und ohne Verschlusssystem miteinbezogen. Als Vorlage für zu entwickelnde bioabsorbierbare Mitralringe aus Mg-Legierungen wurde ein handelsüblicher Mitralring des Typs Carpentier-Edwards classic ausgewählt (dieser besteht hauptsächlich aus nicht resorbierbarem Titan), wobei die unterschiedlichen mechanischen Eigenschaften von Titan und Magnesium bzw. Legierungen mit diesen Metallen als Basislegierungsbestandteil berücksichtigt wurden. Ein handelsüblicher Mitralring wie in Fig. 1 dargestellt wurde vermessen und auf Basis dieser Vermessung ein 3-D-Modell erstellt, das in Fig. 2 dargestellt ist.

Auf Basis des erstellten 3-D-Modells wurde ein Biegewerkzeug hergestellt und es wurden Mitralringe aus einer Magnesiumlegierung aus Stäben mit 2,5 mm Durchmesser und einer Länge von ca. 92 mm bei Raumtemperatur angefertigt. Soweit im Folgenden auf Mg-Mitralringe Bezug genommen wird, handelt es sich dabei um Mitralringe aus einer Magnesiumbasislegierung, also eine Legierung mit Magnesium als Hauptbestandteil.

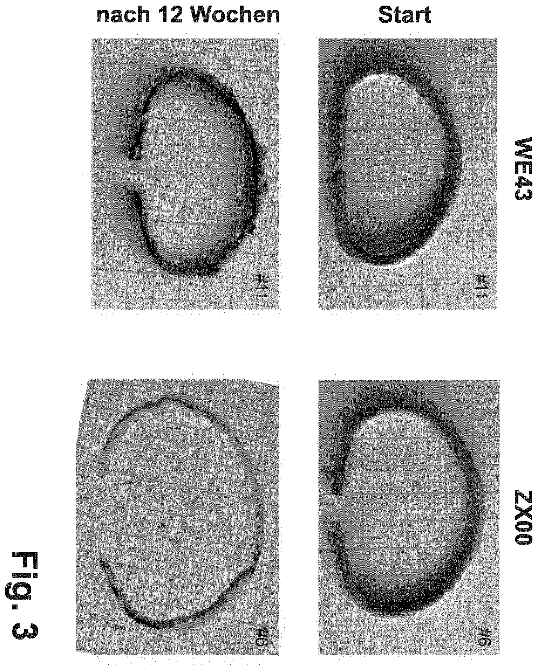

Degradationsanalysen der Mg-Mitralringe in simulierter Körperflüssigkeit (simulated body fluid, kurz SBF) zeigten, dass es nach einigen Wochen zu einem Aufbiegen der wie vorstehend beschrieben hergestellten Mg-Mitralringe kam, wenn diese durch die Degradation dünner wurden. Dies deutet darauf hin, dass durch das Biegen der Stäbchen innere Spannungen in den Mg-Mitralringen aufgebaut werden. Der Zustand entsprechender Mg-Mitralringe zu Beginn einer Degradationsanalyse in SBF und nach 12 Wochen ist für einen Mitralring aus der häufig verwendeten Legierung WE43 sowie der Legierung ZX00 in Fig. 3 dargestellt.

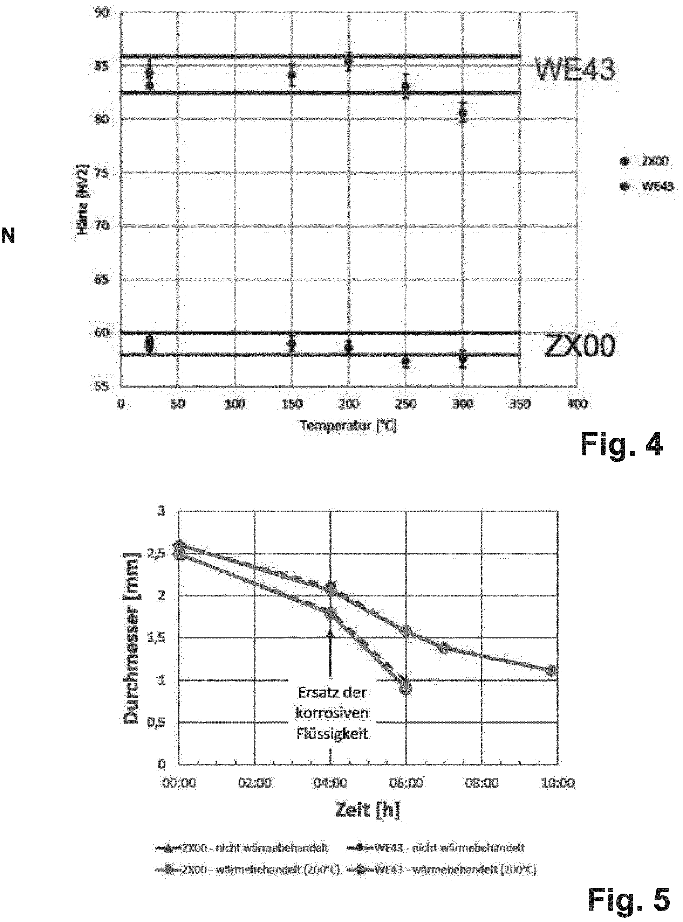

Durch eine geeignet ausgelegte Wärmebehandlung nach dem Biegen der Mg-Mitralringe können innere Spannungen abgebaut werden. Die Wärmebehandlung soll dabei die mechanischen Eigenschaften der Mg-Legierungen nicht verschlechtern. Zur Kontrolle dafür wurde die Härte der wärmebehandelten Mg-Mitralringe bei unterschiedlichen Temperaturen gemessen, wie dies aus Fig. 4 ersichtlich ist. Als optimale Wärmebehandlungstemperatur hat sich bei ZX00 etwa 150° bis 230°C, insbesondere etwa 200 °C, für insbesondere etwa 1h und bei WE43 etwa 250°C für 1h herausgestellt. Damit kann der Effekt des Aufbiegens fast vollständig beseitigt werden. Es wurde auch kein wesentlicher Unterschied auf das Degradationsverhalten der Mg-Mitralringe festgestellt, wie dies in Fig. 5 für die untersuchten Legierungen im Vergleich zwischen wärmebehandelten und nicht wärmebehandelten Mg-Mitralringen ersichtlich ist.

### 2. Entwicklung funktioneller Oberflächenbeschichtungen

Zur Generierung ultra-reiner Nanopartikel aus unterschiedlichen Materialien wurde gepulste Laserablation in Flüssigkeiten eingesetzt. Im ersten Schritt wurden mehrere Lösungsmittel (Dimethylformamid, Tetrahydrofuran [THF], Dichlormethan, Aceton, Chloroform sowie Dimethylsulfoxide) analysiert, um die geeignetsten auszuwählen. Aufgrund eines optimalen Dampfdrucks und geringerer Toxizität wurden Aceton und THF als Lösungsmittel sowie Silber-Nanopartikel (AgNP) als potenziell antimikrobielles Additiv ausgewählt. Der Prozessablauf wurde beginnend mit der Laserablation über die Lagerung der AgNP und die Viskositätseinstellung durch Abdampfen des Lösungsmittels hinsichtlich der Nanopartikelstabilität optimiert; dies trifft auch für eine optimale Menge an dem Lösungsmittel beigemengten Polycaprolacton (PCL) zur Nanopartikelherstellung sowie eine Optimierung der Rate der Nanopartikelsynthese und der Partikelkonzentration in der Polymermatrix zu. Am Ende wurde durch Gießen von AgNP-PCL-Folien eine optische Auswertung (UV-VIS-Absorption) vorgenommen, um die Partikelqualität zu beurteilen. Es konnte mit den Silber-Nanopartikeln eine Dotierung von 3,6 Gew.-% AgNP in PCL erreicht werden. Diese Menge ist laut Literatur allerdings zytotoxisch und wurde daher auf ca. 2 Gew.-% AgNP oder weniger reduziert. Die besten Ergebnisse wurden mit Aceton und mit 1 % PCL gelöst in Aceton erzielt, da es in diesem Lösungsmittel zu einer geringeren Agglomeration von AgNP kam.

Es wurden des Weiteren Syntheseprotokolle für unterschiedliche PCL-Nanopartikel-Komposite wie Silber, Zink-, Kupfer- und Magnesiumnanopartikel entwickelt und Musterchargen erarbeitet. Auch eine direkte Beschichtung der Mg-Mitralringe mit AgNP wurde getestet, die aber weniger zufriedenstellende Ergebnisse lieferte. Daher wurden die antibakteriellen Eigenschaften, welche durch die AgNP hervorgerufen werden, durch Beimischung der AgNP in das PCL, welches zum Elektrospinnen verwendet wird, integriert. Dies bringt den Vorteil, dass kein zusätzlicher Beschichtungsprozess erforderlich ist, welcher die gleichbleibende Qualität der Mg-Mitralringe beeinflussen könnte.

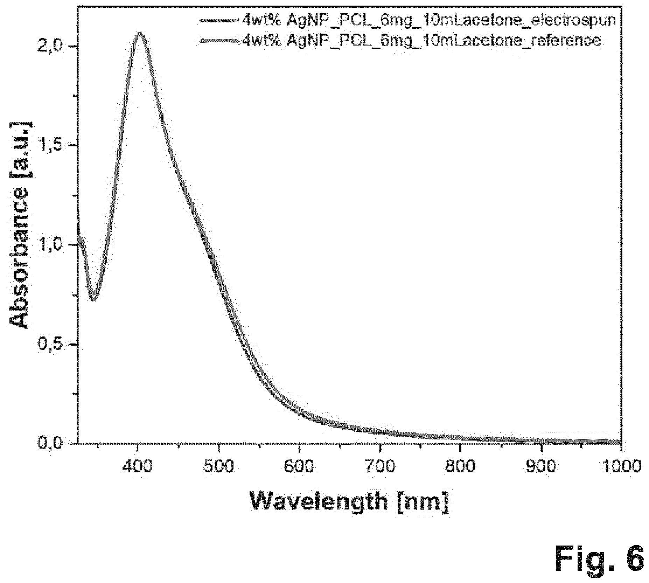

In weiterer Folge wurde getestet, ob die Partikelkonzentration auch noch im elektrogesponnenen Material erhalten geblieben ist, oder ob es Veränderungen gab. Wie aus Fig. 6 ersichtlich ist, zeigen die Ergebnisse von UV-VIS optischen Spektralanalysen, dass die Partikelkonzentration im Granulat und im elektrogesponnenen Material praktisch unverändert ist.

Um die Mg-Mitralringe am Herzen befestigen zu können, wurden mittels eines 3-D-Druckers, der für PCL geeignet ist, Netze mit unterschiedlichen Porengrößen hergestellt. In weiterer Folge wurde aber festgestellt, dass die 3-D-gedruckten Hülsen bereits nach wenigen Wochen keine ausreichende mechanische Qualität und Stabilität mehr aufwiesen, weshalb ein alternatives Verfahren angewendet wurde, um die Halterung für die Mg-Mitralringe herzustellen. Es zeigte sich, dass Elektrospinnen (electrospinning) eine geeignete Methode ist, um Hülsen mit ausreichender mechanischer Stabilität über die erforderlichen Zeiträume herstellen zu können. Die elektrogesponnenen Hülsen bieten nicht nur eine ausreichende mechanische Stabilität und Vernähbarkeit, sondern dienen auch als geeignete funktionelle Basis für eine Einkapselung von antibakteriell wirkenden Nanopartikeln, beispielsweise solche aus Silber.

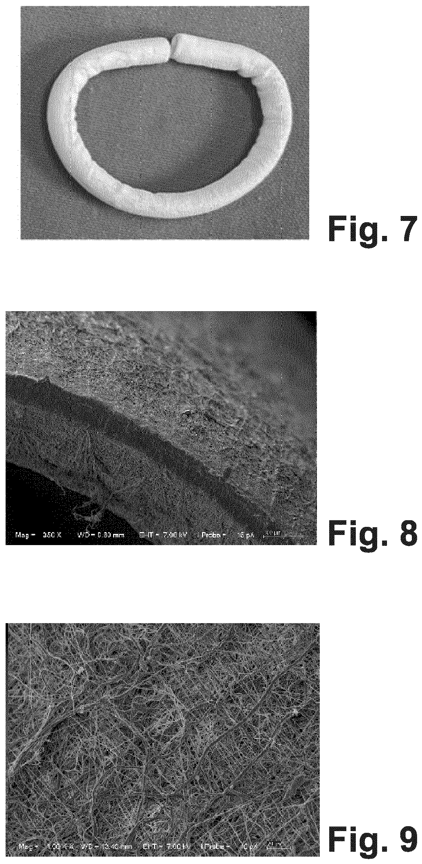

In Fig. 7 ist eine Hülse, die mittels Elektrospinnen hergestellt wurde, dargestellt. In Fig. 8 und Fig. 9 sind hierzu rasterelektronenmikroskopische Aufnahmen gezeigt, aus welchen die Mikrostruktur derartiger Hülsen ersichtlich ist. Auch PCL-Material mit bis zu 3,6 Gew.- % AgNP lässt sich gut elektrospinnen. Für die Herstellung der finalen Netze zur Befestigung der Mg-Mitralringe mittels Elektrospinnen wurde PCL mit 0,5 Gew.-% AgNP dotiertes Granulat hergestellt.

### 3. In-vitro-Tests

### 3.1. Mechanische Tests und Degradationsexperimente

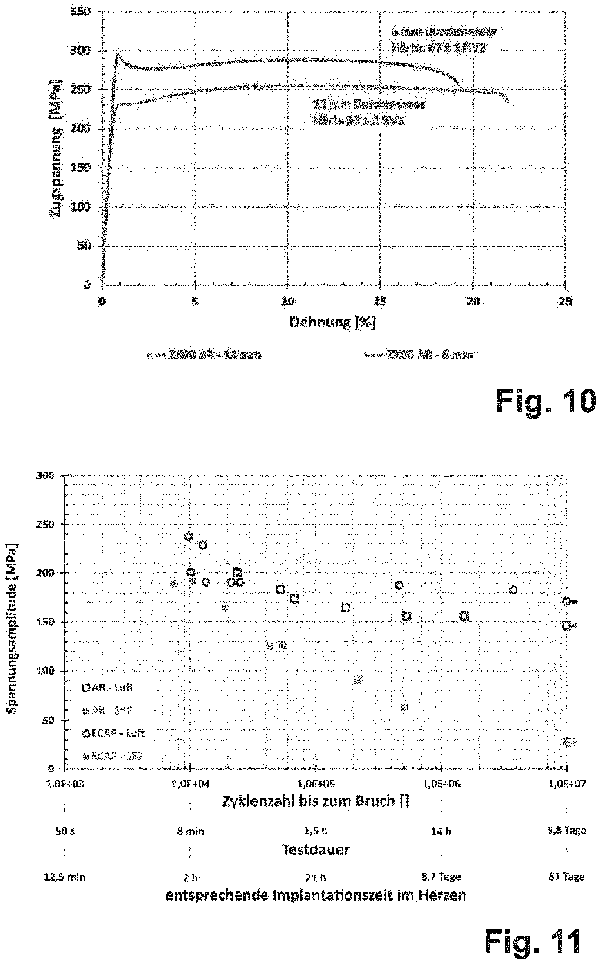

Aus den einschlägigen Fachpublikationen wurden die maximal im Herzen auf einen Mitralring wirksamen Kräfte ermittelt. Typische Kräfte, die auf den Mitralring bei maximalem linksventrikulären Druck von 100 mm Hg wirken, sind (4,9 ± 2,0 N vs. 2,1 ± 1,1 N) bzw. bei 125 mm Hg (5,4 ± 2,3N vs. 2,3 ± 1,2 N) und bei 150 mm Hg (5,7 ± 2,4 N vs. 2,4 ± 1,1 N). Da diese Kräfte im Vergleich zu den Materialkenndaten nach der Extrusion der Mg-Legierungen bzw. nach einer doppelten ECAP-Umformung sehr gering sind, wurde in weiterer Folge extrudiertes Stangenmaterial mit 6 mm Durchmesser anstatt Material mit 12 mm Durchmesser vor und nach einer doppelten ECAP-Umformung für die Herstellung der Mg-Mitralringe sowie von Pins verwendet. Für die biologischen in-vitro-Untersuchungen wurde Stangenmaterial mit einem Durchmesser von 12 mm verwendet. Die Materialkenndaten des extrudierten Stangenmaterials mit Durchmesser von 6 mm im Vergleich zum extrudierten Stangenmaterial mit Durchmesser 12 mm sind in Fig. 10 ersichtlich. Das 6 mm-Material hat eine höhere Zugfestigkeit (290 MPa vs. 230 MPa) und Härte (67 HV2 vs. 58 HV2), aber geringere Duktilität und weniger Kaltverfestigungskapazität als das 12 mm-Material. Daher ist das Material auch schwieriger bei Raumtemperatur durch Biegen in die Form des Mg-Mitralringes zu bringen; teilweise wurden Brüche der Mg-Mitralringe festgestellt. Um Brüche zu vermeiden, können die Mg-Mitralringe bei erhöhten Temperaturen umgeformt und/oder durch Drahtziehen erstellt werden. Beim Drahtziehen kann ein Durchmesser beispielsweise mit 2,5 mm gewählt werden, was zu einem verbesserten Biegeverhalten führt.

In weiterer Folge wurden Ergebnisse von Ermüdungstests in simulierter Körperflüssigkeit bzw. SBF herangezogen, um zu evaluieren, ob der Herzschlag einen negativen Einfluss auf die mechanische Stabilität der Mg-Mitralringe haben könnte. Die Ergebnisse der früheren Versuche sind in Fig. 11 ersichtlich und zeigen, dass eine korrosive Umgebung, wie diese im Körper vorherrscht, die Ermüdungsfestigkeit stark senkt. Unter SBF-Bedingungen zeigte sich ein deutlich geringeres Ermüdungslimit (30 MPa bis 40 MPa) als unter Raumluftbedingungen (145 MPa bis 170 MPa). Dieses Ermüdungslimit ist allerdings immer noch wesentlich höher als die maximale Belastung der Mg-Mitralringe im Herzen und daher ausreichend, um die Mg-Mitralringe an lebenden Objekten zu testen.

### 3.2. 3-Punkt-Biegeversuch an Pins (Ausgangszustand und nach 22 Wochen Implantationszeit)

Um zu beurteilen, wie stabil Pins nach 22 Wochen Implantationszeit in einem subkutanen Rattenmodell sind, wurde ein 3-Punkt-Biegeversuch aufgebaut.

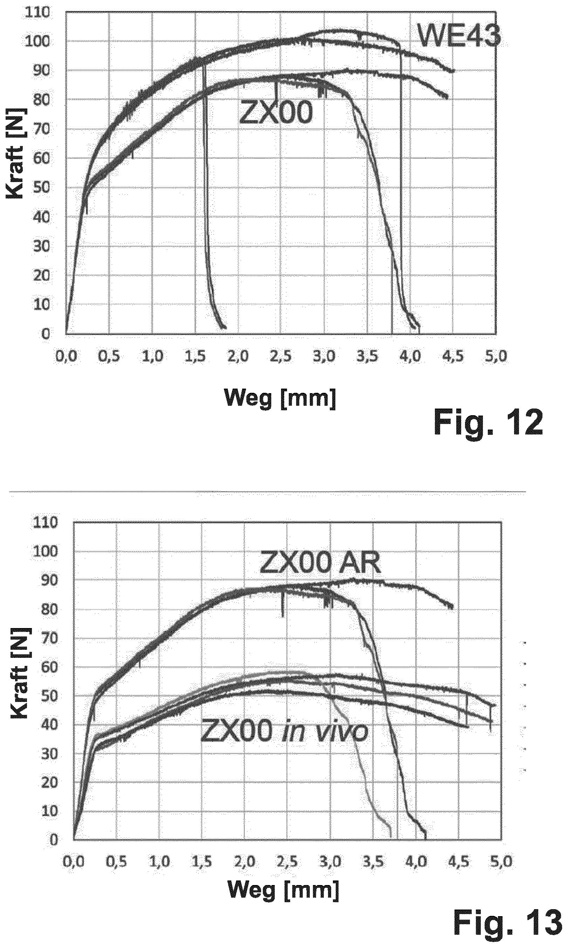

Fig. 12 zeigt die Ergebnisse der 3-Punkt-Biegeversuche mit Pins aus ZX00 und WE43, die nicht implantiert wurden. Gut erkennbar ist, dass Pins aus WE43 aufgrund eines wesentlich höheren Legierungsanteils fester sind, zwei Pins jedoch deutlich früher gebrochen sind als Pins der Legierung ZX00. Eine mittlere maximale Kraft beträgt bei ZX00 89 ± 1 N und bei WE43 98 ± 4 N. Der elastische Bereich ist bei beiden Legierungen ungefähr gleich und erreicht etwa 45 N bis 50 N.

In Fig. 13 sind Ergebnisse von 3-Punkt-Biegeversuchen mit Pins aus der Legierung ZX00 gezeigt, wobei die Pins zuvor 22 Wochen lang implantiert waren. Gut erkennbar ist, dass diese eine geringere Biegefestigkeit aufweisen als die Ausgangspins (-37 %), was auch zu erwarten war, die Duktilität jedoch gleichgeblieben ist bzw. sich sogar leicht verbesserte. Die mittlere maximale Kraft beträgt bei Pins aus der Legierung ZX00 AR (AR entspricht "as received", somit im extrudierten Zustand) 89 N ± 1 N und bei ZX00 in-vivo 56 N ± 3 N. Der elastische Bereich ist bei ZX00 in-vivo geringer bzw. hat abgenommen, liegt aber noch bei ca. 30 N, was immer noch weit über der maximalen Beanspruchung im Herzen liegt.

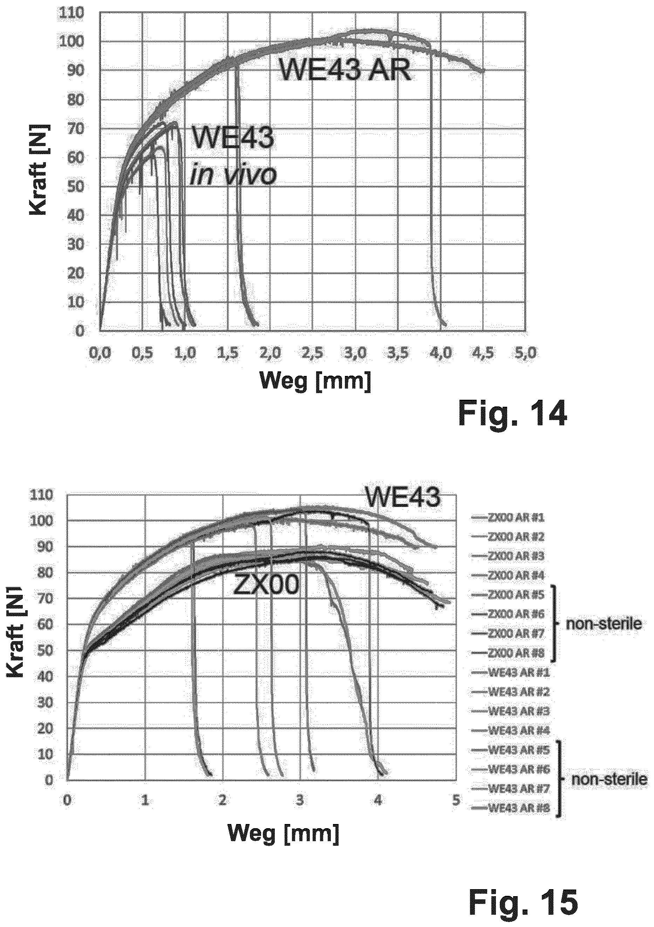

Fig. 14 zeigt Ergebnisse von 3-Punkt-Biegeversuchen an Pins aus der Legierung WE43, wobei die Pins 22 Wochen lang implantiert waren. Gut erkennbar ist, dass die in-vivo getesteten WE43-Pins eine geringere Biegefestigkeit aufweisen (-31 %), was auch zu erwarten war, aber im Unterschied zu den in-vivo-Pins aus der Legierung ZX00 die Duktilität ebenfalls stark abgenommen hatte, was zu einem frühzeitigen mechanischen Versagen führen kann. Die mittlere maximale Kraft beträgt bei Pins aus WE43 AR 98 N ± 4 N und bei Pins aus WE43 in-vivo 68 ± 5 N. Der elastische Bereich ist bei WE43 in-vivo im Vergleich nur wenig geringer und liegt bei ca. 40 N.

Um einen eventuellen Einfluss einer Gammasterilisation auf mechanische Eigenschaften zu prüfen, wurden auch 3-Punkt-Biegeversuche bei nicht-implantierten Pins vor einer Sterilisation durchgeführt. ZX00-Pins hatten vor und nach der Sterilisation den gleichen Kraft-Weg-Kurvenverlauf, wenn auch mit leicht unterschiedlicher Form. Bei WE43-Pins gibt es keinen Unterschied im Kraft-Weg Kurvenverlauf, allerdings kommt es bei den nicht sterilen WE43-Pins öfter zu einem früheren Versagen der Pins. Dies ist aus Fig. 15 ersichtlich.

### 3.3. Einfluss von Reinigung und Sterilisation auf ZX00-Pins

Bei einigen Pins, die nicht für die Implantation benötigt wurden und steril verpackt und sterilisiert gewesen sind, wurde festgestellt, dass an der Oberfläche nach einiger Zeit Korrosionsspuren entstanden sind. Dieser Effekt wurde systematisch untersucht, um die Ursache festzustellen.

Hierfür wurden Plättchen aus der Legierung ZX00 in unterschiedlichen Reinigungsmitteln wie destilliertes Wasser, Isopropanol, Ethanol oder Aceton eingelegt und nach 1h und 4h unter einem Lichtmikroskop untersucht. Es konnten - außer wie erwartet bei destilliertem Wasser - keine Veränderungen der Oberfläche bei den unterschiedlichen Reinigungsmitteln festgestellt werden. Die festgestellten Korrosionsspuren können daher nicht durch einen fehlerhaften Reinigungsprozess der Pins entstanden sein.

Es wurde daher angenommen, dass Umgebungseinflüsse zu diesen Korrosionsspuren geführt haben könnten. Um dies zu untersuchen, wurde ein ZX00-Plättchen einige Tage lang einer Umgebung mit sehr hoher Luftfeuchtigkeit ausgesetzt. Diese Probe wurde dazu zusammen mit einem Schälchen Wasser in einen geschlossenen Plastikbeutel gegeben. Es wurde festgestellt, dass sich bei höherer Luftfeuchtigkeit Korrosionspuren an der Oberfläche bilden, welche den zuvor beobachteten ähnelten. Bei Implantaten, die mechanisch belastet werden, kann diese rissauslösend sein und zu frühzeitigem Versagen führen. Es ist bei der Reinigung und Verpackung von Mg-Implantaten darauf zu achten, dass die Luftfeuchtigkeit möglichst gering ist und das Implantat mit möglichst wenig umgebender Luft verpackt wird.

### 3.4. Degradations- und 3-Punkt-Biegetests an in-vitro degradierten Pins

Es wurde für 5 Wochen lang ein umfangreicher in-vitro-Degradationsversuch in SBF mit Tris/HCl-Puffer an sterilisierten Pins aus den Legierungen ZX00 und WE43 durchgeführt. In jeder Flasche, die mit 250 ml SBF gefüllt war, befanden sich drei bis vier Pins, die ohne Kontakt zur Flaschenwand aufgehängt waren. Alle Flaschen befanden sich in einem Wasserbad, das auf Körpertemperatur (36,5 °C) aufgeheizt war, und waren mit einem Parafilm abgedichtet. Der initiale pH-Wert der SBF lag zwischen 7,35 und 7,45. Die SBF wurde alle sieben Tage gewechselt.

Die Ergebnisse der Degradation zu unterschiedlichen Zeitpunkten (2, 4 und 5 Wochen) wurden visuell festgestellt. Die visuelle Inspektion zeigte, dass die Degradation wie in Vorversuchen festgestellt ablief, das heißt, eine weiße Oberfläche bei den ZX00-Pins und schwarze Oberfläche mit abfallenden schwarzen Partikeln bei den WE43-Pins erhalten wurde. Allerdings ist die Degradation rascher und mit einer größeren Varianz von Pin zu Pin erfolgt als erwartet wurde, weshalb der Versuch bereits nach fünf Wochen beendet wurde.

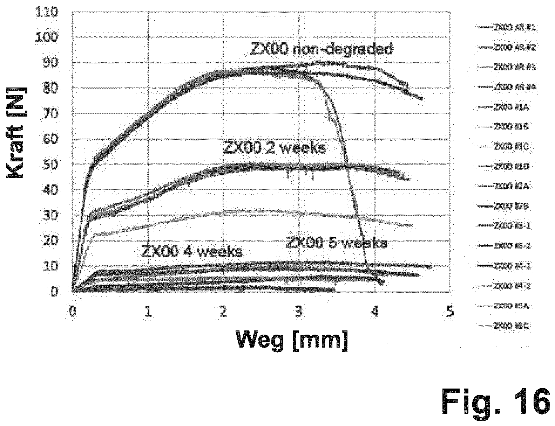

An den in-vitro degradierten Pins wurden auch 3-Punkt-Biegeversuche durchgeführt, um das mechanische Verhalten zu verschiedenen Zeitpunkten der Degradation evaluieren zu können. Die Ergebnisse sind für ZX00-Pins in Fig. 16 ersichtlich und können wie folgt zusammengefasst werden: Die Festigkeit hat mit zunehmender Degradationszeit abgenommen, während die Duktilität bis Woche 4 fast unverändert geblieben ist. Keiner der Pins ist beim Versuch gebrochen, aber es gab nach 5 Wochen eine größere Streuung bei den Kraft-Weg-Kurven.

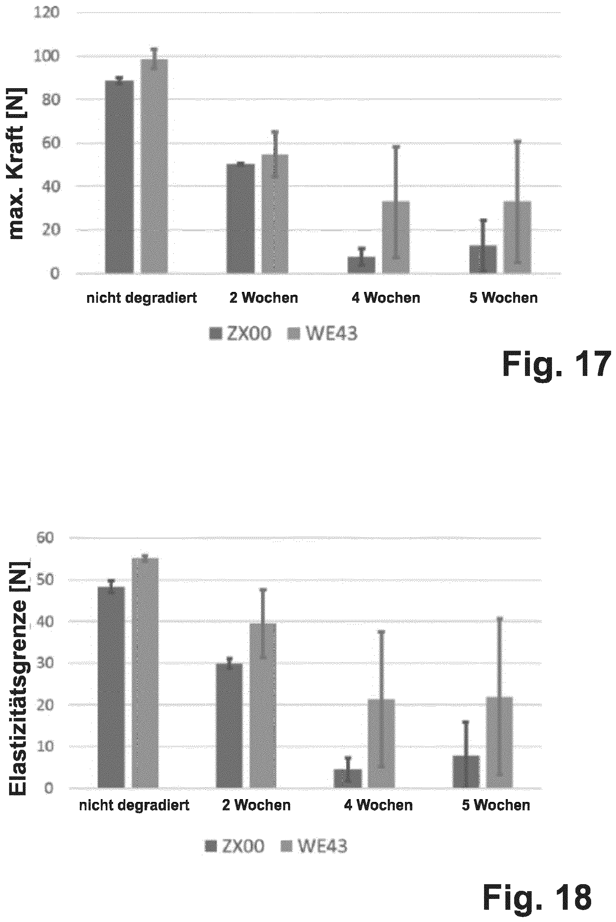

Die Auswertung und Zusammenfassung der maximalen Kräfte und der Elastizitätsgrenze aus den Kraft-Weg-Kurven ist in Fig. 17 und Fig. 18 dargestellt. Die ZX00-Pins haben eine geringere Festigkeit als die WE43-Pins sowohl im nicht degradierten Zustand als auch nach unterschiedlichen Stadien der Degradation. Die WE43-Pins zeigen aber eine höhere Variation bei den Festigkeitswerten bereits nach 2 Wochen, aber speziell nach 4 und 5 Wochen. Eine hohe Streuung ist bei Implantaten unerwünscht, sodass auch aus diesem Grund die ZX00-Legierung der WE43-Legierung vorzuziehen ist. Die elastische Verformung verläuft analog zur maximalen Kraft bzw. Festigkeit bei den beiden Legierungen und ist in Fig. 18 ersichtlich.

### 3.5. Biologische Tests - Biokompatibilität

Für Biokompatibilitätstests des Materials der Hülsen aus PCL (80 kDa) mit unterschiedlicher Dotierung mit Silber-Nanopartikeln (AgNP) wurden Proben angefertigt. Es wurde ein methodischer Ansatz geplant, der auf einem adaptierten Testprotokoll zur Bestimmung der antibakteriellen Aktivität auf Kunststoffoberflächen und anderen nichtporösen Oberflächen basierend auf der Norm ISO 22196:2011 (Kunststoffmaterialien) beruht. Dieses Protokoll beruht auf einer Oberflächen- anstatt einer Masseprüfung. Als Bakterienstämme wurden *Escherichia coli* und *Staphylococcus aureus* ausgewählt. Für eine Zytotoxizitätsprüfung wurden elektrogesponne Netze auf sogenannte CellCrowns montiert (erhältlich unter www.scaffdex.com).

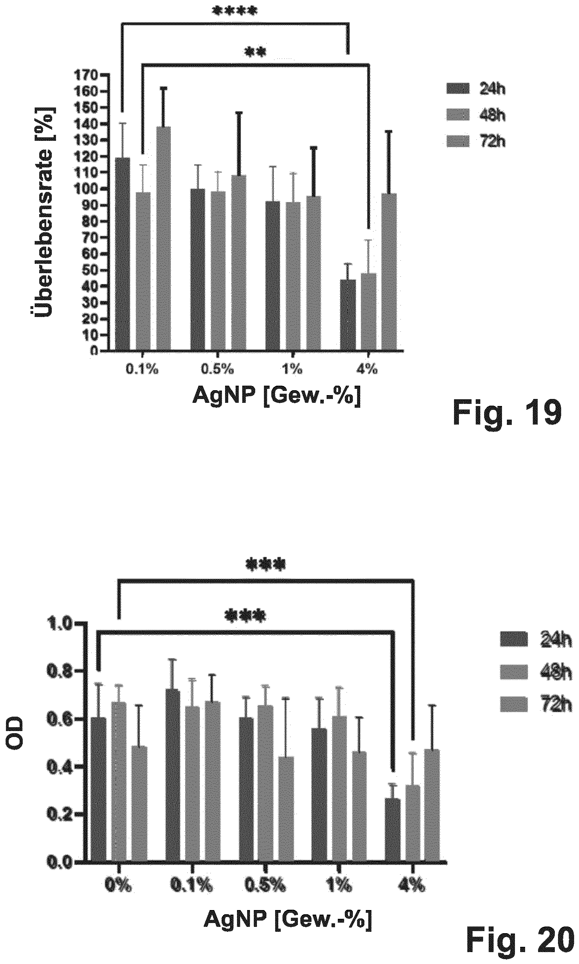

Eine Zytotoxizitätsprüfung der elektrogesponnenen PCL-Ag Nanocomposites mittels XTT-Tests hat ergeben, dass es bei einer Konzentration von 4 Gew.-% AgNP-Dotierung zu einem signifikanten Abfall der Lebensfähigkeit der Zellen kommt (siehe Fig. 19 und Fig. 20). In den niedrigeren Konzentrationen wurden keine signifikanten Unterschiede der Lebensfähigkeit der Zellen festgestellt. Deshalb wurde in weiterer Folge auf die Konzentrationen 0,1 Gew.-%, 0,5 Gew.-% und 1 Gew.-% fokussiert.

Zur finalen Bestimmung der Konzentration der Silber Nanopartikeldotierung von PCL für die Netze der Mg-Mitralringe wurden folgende AgNP-dotierten PCL-Ausgangsmaterialien für das Elektrospinnen verwendet: 0 Gew.-% AgNP, 0,1 Gew.-% AgNP, 0,5 Gew.-% AgNP und 1 Gew.-% AgNP. Die Ergebnisse der Bestimmung der antibakteriellen Aktivität in einem zertifizierten Prüflabor sind in folgender Tabelle für verschieden dotiertes PCL sowie für beide Bakterienstämme (*E*. *coli* und *S*. *aureus*) dargestellt. Es zeigte sich, dass in der Konzentration von 0,5 Gew.-% AgNP dotiertes PCL einen Log-Reduction-Faktor R von > 2 für ausreichend antibakterielle Effektivität bei beiden Bakterienstämmen erreichte (siehe nachstehende Tabelle 1). Diese Konzentration wurde daher verwendet, um die finalen Netze für die Mg-Mitralringe herzustellen.

**Tabelle 1: Antibakterielle Aktivität R getesteter Proben**

| **Probe** | **Testbakterium** | **Antibakterielle Aktivität R** | |
|---|---|---|---|
| | | **Reduktion [log]** | **Reduktion [%]** |
| PCL elektrogesponnen ohne Nanopartikel | *S*. *aureus* DSM 346 | 0,2 | 31,6 |
| | *E. coli* DSM 1576 | 0,3 | 49,8 |
| PCL elektrogesponnen mit 0,1 Gew.-% AgNP | *S*. *aureus* DSM 346 | 0,9 | 88,2 |
| | *E. coli* DSM 1576 | 5,1 | 99,9992 |
| PCL elektrogesponnen mit 0,5 Gew.-% AgNP | *S*. *aureus* DSM 346 | 2,1 | 99,3 |
| | *E. coli* DSM 1576 | 5,4 | 99,9996 |
| PCL elektrogesponnen mit 1 Gew.-% AgNP | *S*. *aureus* DSM 346 | 4,4 | 99,996 |
| | *E. coli* DSM 1576 | 4,8 | 99,998 |

### 3.6. In-vivo-Tests

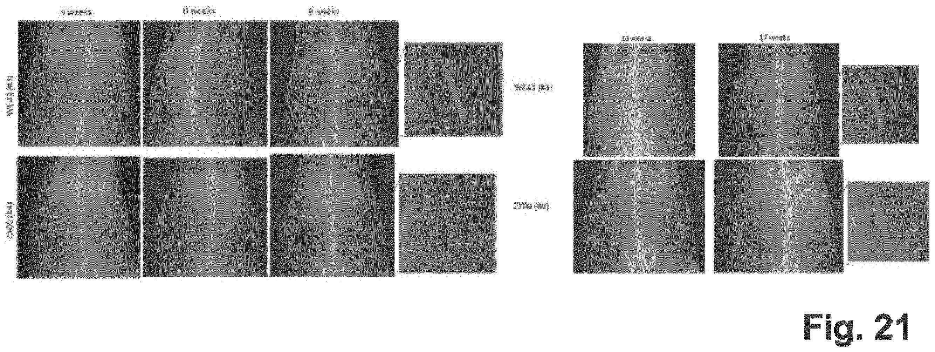

Ein Ethikantrag zur Durchführung einer Kleintierstudie wurde eingebracht und genehmigt. Es handelt sich um Verträglichkeitsstudien, bei welchen subkutan die einzelnen Bestandteile (Mg-Pin, Hülse sowie der gesamte Mg-Mitralring) in aufeinanderfolgenden Phasen implantiert werden. Diese präklinischen Verträglichkeitsstudien wurden zunächst mit Ratten durchgeführt. In den Ratten wurden Mg-Pins subkutan auf dem Rücken implantiert. Es wurden die beiden Mg-Legierungen ZX00 und WE43 für die Pins verwendet und bei insgesamt acht Ratten jeweils vier Pins vom gleichen Mg-Material implantiert. In den ersten zwei Wochen wurden mehrmals Röntgenbilder aufgenommen, um das Degradationsverhalten und eventuelle Blasenbildungen sowie die einseitige Fixierung der Implantate zu monitoren. Danach wurden einmal wöchentlich Röntgenaufnahmen durchgeführt, die in Fig. 21 dargestellt sind. Es konnte keine Gasblasenbildung festgestellt werden und alle Pins, bis auf einen WE43-Pin, sind bis in die Woche 17 stabil am Implantationsort verblieben. Dieser eine Pin wurde offensichtlich durch eine andere Ratte entfernt, da Nähte aufgerissen waren. Es konnte keine messbare Degradation im Röntgen in den ersten 17 Wochen festgestellt werden. Alle Tiere waren in einem guten gesundheitlichen Zustand. Die Implantationszeit wurde verlängert. Die Explantation der Pins erfolgte in Woche 22 nach der Implantation der Pins.

Aufnahmen der Pins bei der Explantation von WE43-Pins bei Ratte Nr. 1 und von ZX00-Pins bei Ratte Nr. 2 nach 22 Wochen Implantationszeit zeigten die folgenden Merkmale: In-situ gab es keine groben Anzeichen von Degradation bei den Pins. Dies bestätigt die Aufnahmen durch Röntgenbilder. Es gab auch keine sichtbaren Anzeichen von Entzündungen. Die Implantate waren von einer dünnen Gewebekapsel umgeben. Nur bei einem ZX00-Pin waren sehr kleine Gasblasen ersichtlich. Bei den WE43-Pins wurde bei der Explantation beobachtet, dass das Gewebe weniger anhaftet als dies bei den ZX00-Pins der Fall ist.

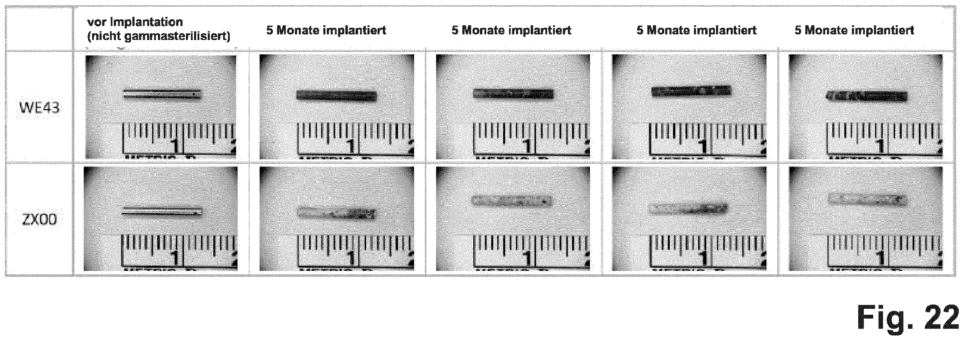

In Fig. 22 sieht man stereomikroskopische Aufnahmen von explantierten WE43- und ZX00-Pins. Die Bilder wurden nach einer 24h-Fixierung durch 2,5 % Glutaraldehyd zur Konservierung der biologischen Gewebeproben an der Oberfläche aufgenommen. Danach wurden die explantierten Pins trocken gelagert, um eine weitere Degradation zu verhindern. Es gab keine Längenveränderung der Pins. Die WE43-Pins hatten eine sehr einheitliche schwarze Oberfläche im Vergleich zu einer heterogenen mehrheitlich weißen Oberfläche bei den ZX00-Pins. Bei ZX00-Pins gab es kleinere lokalisierte Korrosions-Hot-Spots. Der Gewichtsvergleich der WE43- und ZX00-Pins vor der Implantation und nach 22 Wochen Implantationszeit hat folgende Werte ergeben: WE43 vor der Implantation 43,0 mg und nach 22 Wochen 42,2 mg bis 42,8 mg, ZX00 vor der Implantation 40,0 mg und nach fünf Wochen 36,7 mg bis 39,1 mg. Das unterschiedliche Gewicht vor der Implantation ist dadurch erklärbar, dass die WE43-Legierung schwere Seltenen Erden Elemente enthält. Erkennbar ist auch, dass der Gewichtsverlust bei den ZX00-Pins größer ist als bei den WE43-Pins. Dies war bereits bei den in-vitro-Versuchen der Fall.

Lichtmikroskopische Bilder der explantierten ZX00-Pins nach 22 Wochen zeigten, dass die Pins einschließlich der Degradationsschicht in deren Durchmesser bei reiner optischer Betrachtung fast gleichgeblieben sind. Das Loch, wo sich die Bohrung zur Befestigung der Pins befindet, wurde bei manchen Pins größer. Die Degradationsschicht sieht bei den ZX00-Pins wesentlich anders aus als bei WE43-Pins und ist auch wesentlich dicker. Die Degradationsschicht bei den ZX00-Pins sieht teilweise wie eine organische Oberfläche aus und reicht farblich von grau zu weiß bis gelb.

WE43-Pins weisen nach 22 Wochen Implantationszeit wie vor dem Einsetzen eine schwarze Oberfläche auf, an der teilweise noch die Bearbeitungsspuren erkennbar sind. Der Durchmesser scheint sich kaum verändert zu haben und an einigen Stellen sind pockenartige Strukturen erkennbar, die Degradationsprodukte sein könnten. In einer höheren Auflösung sind in der Degradationsschicht viele kleine Risse erkennbar und an manchen Stellen ist diese Schicht abgefallen, wodurch Dellen von ca. 20 bis 25 µm Tiefe entstanden sind.

Die bereits lichtmikroskopisch untersuchten Pins wurden anschließend in 3-Punkt-Biegeversuchen hinsichtlich ihrer mechanischen Stabilität getestet. Die Ergebnisse sind vorstehend dargestellt.

Das venöse Blut der Ratten wurde untersucht. Zwischen den beiden Implantat-Materialien sind keine signifikanten Unterschiede feststellbar. Die Leberwerte Alkalische Phosphatase (AP), Aspartat-Aminotransferase (AST) und Alanin-Aminotransferase (ALT) waren im Vergleich zu unbehandelten gesunden Tieren gemäß den Referenzwerten in der Publikation "Reference values for selected hematological, biochemical and physiological parameters of Sprague-Dawley rats at the Animal House, Faculty of Medicine, University of Colombo, Sri Lanka, Shehani L. Delwatta et.al., Animal Model Exp Med. 2018*"* im normalen Wertebereich. Nur die AP-Werte bei WE43 und ZX00 lagen unterhalb des Grenzwertes, wobei ZX00 etwas näher am Grenzwert liegt als WE43. Die Nierenwerte Kreatinin und Harnstoff (BUN) sind ebenfalls im normalen Wertebereich, wobei der BUN-Wert bei beiden Legierungen im oberen Grenzwertbereich liegt. Im Blutbild und bei den Magnesium Werten im Blut konnten keine signifikanten Unterschiede zwischen den beiden Mg-Legierungen festgestellt werden. Im Blutbild und bei den Magnesiumwerten im Blut konnten keine signifikanten Unterschiede zwischen den beiden Mg-Legierungen festgestellt werden. Die histologischen Bilder bestätigen eine angemessene Integration in das angrenzende Gewebe mit einer geringen Entzündungsreaktion. Außerdem waren die Degradation sehr homogen und die Gasproduktion bei den meisten Implantaten sehr gering und konstant.

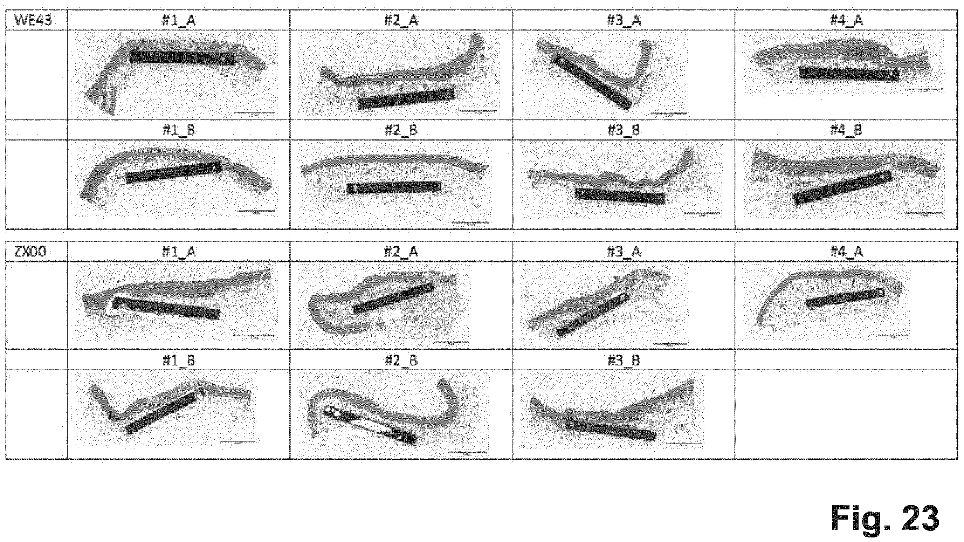

Es wurden histopathologische Analysen der Implantat-Umgebung vom subkutanen Gewebe vorgenommen und eine hierfür übliche Färbung des entnommenen Gewebes durchgeführt und es konnten keine Entzündungen im Hautgewebe festgestellt werden (Fig. 23).

Die bei umfangreichen histopathologischen Analysen an den Organen Gehirn, Herz, Lunge, Leber, Niere, Lymphknoten und Milz festgestellten mikroskopischen Befunde waren meist geringfügig und gehören zu den spontanen Hintergrundbefunden, die bei unbehandelten Ratten in diesem Alter auftreten. Die histopathologischen Analysen haben daher keine Auffälligkeiten im Vergleich zu gesunden Ratten ergeben, sodass bei beiden Mg-Legierungen von einer guten Verträglichkeit über einen Zeitraum von 22 Wochen und mehr ausgegangen werden kann.

### 3.7. Akutversuche an Schweinen und Implantation bei Schafen

Der Akutversuch an Schweinen wurde durchgeführt, um einerseits die chirurgische Vernähbarkeit der elektrogesponnenen Netze in-vivo zu bestätigen, und um andererseits die mechanische Stabilität des Mg-Mitralrings aus ZX00 mit einem PCL-Netz bei der Operation und kurz danach zu prüfen. Die Analyse der explantierten Mg-Mitralringe nach dem Akutversuch mit den elektrogesponnenen Netzen zeigte, dass beide Anforderungen, Fixierung der Mg-Mitralringe im Herzen und die Operabilität der Mg-Mitralringe mit PCL-Netz, sehr gut erfüllt wurden.

Für ein Studienprotokoll bei der Schafstudie wurde eine Kontrollgruppe von zwei Schafen mit einem Standard-Mitralring, kommerziell erhältlich von Edwards, definiert und eine Gruppe von fünf Schafen mit dem finalen Mg-Mitralring mit einer PCL-Ummantelung, welche mit 0,5 Gew.-% AgNP dotiert ist, gewählt. Die Mg-Mitralringe, die wärmebehandelt waren, wurden zunächst mit Isopropanol in einem Ultraschallbad gereinigt und mit einem elektrogesponnenen PCL-Material welches mit 0,5 Gew.-% AgNP dotiert war, überzogen. Diese Mg-Mitralringe wurden in einer Sterilverpackung verpackt und gammasterilisiert.

Die Implantation der Mg-Mitralringe aus der ZX00-Legierung bei fünf Schafen verlief ohne größere Probleme, obwohl die Operationstechnik bei den Schafen am schlagenden Herzen einige Herausforderungen mit sich brachte. Die fünf Schafe erholten sich rasch nach der Operation und es waren in der Nachuntersuchungsperiode keine klinischen Auffälligkeiten feststellbar. Mit laufend durchgeführten Ultraschalluntersuchungen wurde die ausreichende Stabilität der erfindungsgemäßen Implantate und deren Abbau laufend kontrolliert. Weiter wurde mittels Blutbild- und Blutchemieanalysen der postoperative Zustand der Tiere überwacht, wobei bei allen Tieren sehr zufriedenstellende Ergebnisse erzielt wurden.

## Patentansprüche

1. Implantat zur medizinischen Versorgung von Menschen und/oder Tieren, umfassend oder bestehend aus:
a) einem bioresorbierbaren Implantatgrundkörper;
b) einem bioresorbierbaren Befestigungskörper, der mit dem Implantatgrundkörper in Verbindung steht und über welchen der Implantatgrundkörper in einem menschlichen oder tierischen Körper befestigbar ist;
c) eine antibakteriell wirkende Substanz.

2. Implantat nach Anspruch 1, wobei der Implantatgrundkörper aus einem Metall, einem Verbundwerkstoff mit überwiegend metallischen Anteilen oder insbesondere einer Metalllegierung gebildet ist.

3. Implantat nach Anspruch 1 oder 2, wobei der Implantatgrundkörper aus einer Magnesiumlegierung gebildet ist.

4. Implantat nach Anspruch 3, wobei die Magnesiumlegierung in Massenprozent folgende Elemente aufweist oder aus diesen besteht:
0,2 % bis 1,0 %, bevorzugt 0,3 % bis 0,8 %, insbesondere 0,5 % bis 0,7 %, Zink;
0,2 % bis 1,0 %, bevorzugt 0,3 % bis 0,7 %, insbesondere 0,4 % bis 0,6 %, Calcium; optional weitere Legierungselemente wie Titan, Bor oder Silicium im Ausmaß von insgesamt weniger als 0,1 %, bevorzugt weniger als 0,05 %;
Rest Magnesium und herstellungsbedingte Verunreinigungen.

5. Implantat nach Anspruch 3 oder 4, wobei die Magnesiumlegierung mit Ausnahme von herstellungsbedingten Verunreinigungen frei von Metallen der Seltenen Erden ist.

6. Implantat nach einem der Ansprüche 1 bis 5, wobei der Implantatgrundkörper ein Annuloplastiering ist, beispielsweise ein Mitralring, insbesondere ein offener Mitralring.

7. Implantat nach einem der Ansprüche 1 bis 6, wobei der Befestigungskörper aus einem Polymer gebildet ist.

8. Implantat nach einem der Ansprüche 1 bis 7, wobei der Befestigungskörper aus einem Polymer gebildet ist, das durch Elektrospinnen abgeschieden ist.

9. Implantat nach einem der Ansprüche 1 bis 8, wobei die antibakteriell wirkende Substanz Nanopartikel umfasst oder aus diesen besteht.

10. Implantat nach einem der Ansprüche 1 bis 9, wobei die antibakteriell wirkende Substanz im und/oder am Befestigungskörper verteilt ist, insbesondere homogen verteilt ist.

11. Implantat nach Anspruch 10, wobei die antibakteriell wirkende Substanz in Massenprozent einem Anteil von 0,10 % bis 6,0 %, bevorzugt 0,20 % bis 4,5 %, insbesondere 0,30 % bis 4,0 %, beispielsweise 0,50 % bis 1,5 %, bezogen auf die Gesamtmasse von antibakteriell wirkender Substanz und Befestigungskörper, vorliegt.

12. Bioresorbierbares Verbundmaterial für ein Implantat, das im menschlichen oder tierischen Körper implantiert wird, bestehend aus oder umfassend eine bioresorbierbare Trägersubstanz und eine antibakteriell wirkende Substanz, wobei das Verbundmaterial elektrogesponnen ist und/oder als Beschichtung vorliegt.

13. Bioresorbierbares Verbundmaterial nach Anspruch 12, wobei die Trägersubstanz bei Temperaturen von weniger als 100 °C aufschmelzbar ist.

14. Bioresorbierbares Verbundmaterial nach Anspruch 12 oder 13, wobei die antibakteriell wirkende Substanz in Massenprozent einem Anteil von 0,10 % bis 6,0 %, bevorzugt 0,20 % bis 4,5 %, insbesondere 0,30 % bis 4,0 %, beispielsweise 0,50 % bis 1,5 %, bezogen auf die Gesamtmasse von antibakteriell wirkender Substanz und Trägersubstanz, vorliegt.

15. Bioresorbierbares Verbundmaterial nach einem der Ansprüche 12 bis 14, wobei die antibakteriell wirkende Substanz Nanopartikel umfasst oder aus diesen besteht.
